# EUROPEAN PATENT SPECIFICATION

(11) **EP 4 178 321 B1**
(45) Date of publication and mention of the grant of the patent: **23.10.2024**
(21) Application number: 22205002.3
(22) Date of filing: 02.11.2022
(51) Int. Cl.: H05B 47/115, H05B 47/20

(54) **DEVICE AND METHOD FOR CONTROLLING A LIGHTING AND SANITIZING SYSTEM**
VORRICHTUNG UND VERFAHREN ZUR STEUERUNG EINES BELEUCHTUNGS- UND DESINFEKTIONSSYSTEMS
DISPOSITIF ET PROCÉDÉ DE COMMANDE D'UN SYSTÈME D'ÉCLAIRAGE ET DE DÉSINFECTION

(30) Priority: 04.11.2021 IT 202100028142
(43) Date of publication of application: 10.05.2023
(73) Proprietor: ARTEMIDE S.p.A., 20122 Milano (IT)
(72) Inventor: DE BEVILACQUA, Carlotta Francesca Isolina Maria, 20100 Milano (IT); GALLUCCI, Andrea, 20122 Milano (IT)
(74) Representative: Studio Torta S.p.A.

(56) References cited:
- US-A1- 2007 085 487
- US-A1- 2021 322 597

## Description

### CROSS-REFERENCE TO RELATED APPLICATIONS

This patent application claims priority from Italian Patent Application no. 102021000028142 filed on November 4, 2021.

### TECHNICAL FIELD

The present invention relates to a device and method for controlling a lighting system which integrates lighting and sanitizing (disinfectant, antibacterial, etc.) functions.

### BACKGROUND

The use of UV lamps (in particular, UV lamps emitting in the UV-C band) with sanitizing function is well known, UV light being capable of deactivating or eliminating pathogenic microorganisms.

On the other hand, UV-C light is not suitable for lighting environments and also has potentially dangerous effects on humans too, if not properly controlled.

It is also known that certain light radiations in the visible range, particularly in the violet range, can have disinfectant effects. Lighting devices are therefore known which include a light source emitting specific-wavelength violet light with disinfectant function combined with a light source emitting a complementary light resulting in white light.

US2021322597A1 discloses a UV disinfection system including a UV lamp and a UV controller; the controller is configured to deactivate the UV lamp based on data from a remote sensor being indicative of a detected presence of a life form.

US2007085487A1 discloses an inductively coupled ballast circuit that can be used to control various devices and lamps, for example a UV water treatment system. In some embodiments, the ballast circuit provides feedback to a control unit driving an ultraviolet lamp, so that the control unit can determine if the ultraviolet lamp is on or off and also, in other embodiments, the amount of current and voltage being applied to the ultraviolet lamp.

In general, well-known lighting devices that are also equipped with UV-C sources and/or violet light sources with sanitizing function are not fully satisfactory.

In particular, the problem arises of a safe control of potentially hazardous emissions for users. In fact, not only UV radiation, but also violet light having a wavelength suitable for the sanitizing function can be harmful, especially if users are exposed for long periods and/or at high intensity.

### SUMMARY

The object of the present invention is to overcome the drawbacks of the prior art, in particular by providing a device and a method for controlling a lighting and sanitizing system which is simple and reliable and fully efficient and safe.

The present invention therefore relates to a device and a method for controlling a lighting and sanitizing system as defined in appended claims 1 and 10, respectively.

The invention provides a device and method for controlling a lighting and sanitizing system, which is simple and reliable, and fully efficient and safe. In particular, the control device and method of the invention ensure that the sanitizing source, potentially dangerous to users, is only activated under full safety conditions.

### BRIEF DESCRIPTION OF THE DRAWINGS

Further features and advantages of the present invention will be apparent from the description of the following non-limiting embodiment with reference to the figures of the accompanying drawings, wherein:
- Figure 1 is a schematic view of a lighting and sanitizing system equipped with a control device according to the invention;
- Figure 2 is a schematic view of the control device of the invention.

### DESCRIPTION OF EMBODIMENTS

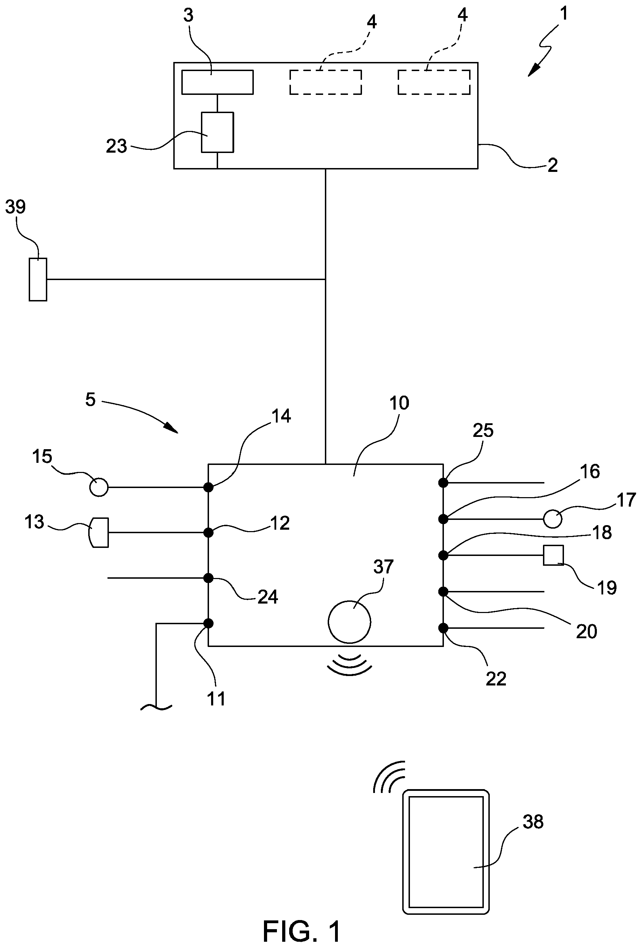

The numeral 1 in Figure 1 shows, as a whole, a lighting system with lighting and sanitizing functions, for example, operating in an environment (one or more rooms).

The lighting system 1 comprises at least one lamp 2 including at least one sanitizing source 3, which emits radiation capable of performing a sanitizing function, such as for example UV radiation, in particular UV-C, or violet light having a wavelength between 380 and 430 nm.

The lamp 2 may include one or more sanitizing sources 3, even different ones (e.g., one or more UV sources and one or more violet light sources); optionally, the lamp 2 may also include one or more visible light sources 4 emitting white light, alone and/or in combination with respective violet light sources.

The lighting system 1 may comprise a plurality of lamps 2, even different ones, i.e., equipped with different sanitizing sources 3 and/or different visible light sources 4.

For example (but not necessarily), the lighting system 1 comprises one or more lamps of the type described in Italian Patent Application no. 102020000010399.

For the sake of simplicity, reference will be made hereinafter to a lighting system 1 with a single lamp 2 equipped with one sanitizing source 3, but it is understood that the invention can also be implemented with several lamps 2 and/or several sanitizing sources 3.

The lighting system 1 comprises a control device 5 connected to the lamp 2 and controlling the lamp 2, and in particular the sanitizing source 3, in order to avoid health risks to users who may be exposed to the emission from the sanitizing source 3.

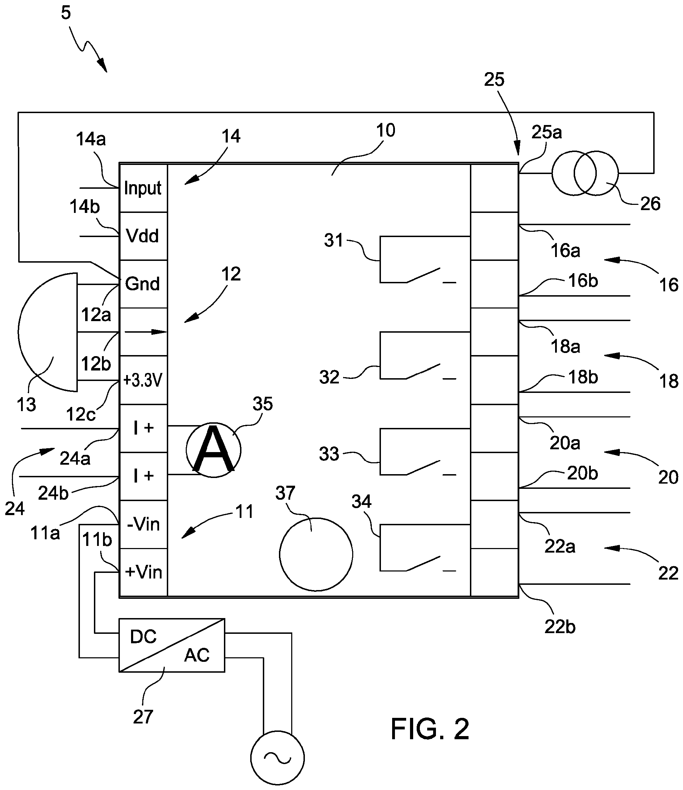

Also referring to Figure 2, the control device 5 comprises, in particular, a printed circuit board 10, comprising:
a supply input 11 for connection to a power supply, for example a 12-48 V direct current power supply;
a sensor input 12 connected to a presence sensor 13, for example a PIR sensor (passive infrared sensor);
an outer sensor input 14 for connection to further outer sensors 15, e.g., one or more further presence sensors and/or door opening sensors and/or a consent sensor (which can be activated, for example, by means of a key or an unlock code);
a first relay contact output 16 for connection to a visual signalling LED 17;
a second relay contact output 18 for connection to a sound signal 19 (a buzzer);
a third relay contact output 20 for sanitization in progress;
a fourth relay contact output 22 for connection in series to a driver 23 of the sanitizing source 3;
a current measure input 24 for measuring the current in the sanitizing source 3;
a transformer input 25 for connection to an amperometric transformer 26 for measuring the primary current in the driver 23 of the sanitizing source 3.

The supply input 11 comprises, for example, a pair of supply terminals 11a, 11b connected to a power supply 27.

The sensor input 12 comprises three terminals 12a, 12b, 12c, including an earth terminal 12a, which are connected to the presence sensor 13. The presence sensor 13 is placed in the room where the lighting system 1 is located to detect the presence of people in this room.

The outer sensor input 14 comprises a pair of terminals 14a, 14b connected to the sensors 15, in particular one or more door opening sensors acting on respective doors of the room in which the lighting system 1 operates, to detect the opening of these doors.

The first output 16 is the output of a first relay 31, in particular a low voltage (max 50V, 100mA) static relay, and comprises a pair of terminals 16a, 16b connected to the visual signalling LED 17, which is placed in an appropriate position to send a visual signal (steady or flashing light) to users.

The second output 18 is the output of a second relay 32, in particular a low voltage (max 50V, 100mA) static relay, and comprises a pair of terminals 18a, 18b connected to the sound signal 19, which is placed in an appropriate position to send a sound signal to users.

The third output 20 is the output of a third relay 33 to signal sanitization in progress (e.g., max 250V, 3A), and comprises a pair of terminals 20a, 20b to indicate that the sanitizing source 3 is switched on and sanitization is in progress.

The fourth output 22 is the output of a fourth relay 34 (e.g., max 250V, 3A) connected in series to the driver 23 of the sanitizing source 3 and comprises a pair of terminals 20a, 20b.

The current measure input 24 comprises a pair of terminals 24a, 24b connected to the sanitizing source 3 to measure, via an amperometric sensor 35, the current to the lamp load (secondary driver), i.e., the current in the sanitizing source 3 on the secondary side of the respective driver 23.

The transformer input 25 comprises a terminal 25a connected to the transformer 26 for measuring the primary current in the driver 23 of the sanitizing source 3. The other wire of the transformer 26 is inserted in the earth terminal 12a.

The control device 5 is then provided with a communication system 37, preferably a wireless communication system, for example Bluetooth, to communicate, other than with the presence sensor 13 and the other sensors 15, with a remote device 38, for example, a smartphone or other similar device loaded with a dedicated application (app) providing a user interface with the control device 5.

The operation of the control device 5 and thus of the lighting system 1, for implementing the method according to the invention, is as follows.

Via the app (and therefore via the device 38) the user can select various functions, in particular:
- safety chain operation test: the app activates a command setting the control device 5 into test mode. In this mode, if the presence sensor 13 and/or one of the other sensors 15 (e.g., a door opening sensor) detects a presence or the opening of a door:
   1) the first output 16 is activated and the visual signalling LED 17 switches on; in this way, a visual test is carried out for the correct functioning of the safety chain, which activates instantaneously if users are present; and
   2) the second output 18 is activated and the sound signal 19 switches on; in this way, a sound test is carried out for the correct functioning of the safety chain, which activates instantaneously if users are present;
- activation of sanitization: either via the app or via a switch 39 (e.g., a bll-type switch), the sanitizing source 3 (or the entire lamp 2) is switched on; the control device 5 provides the consent to switch on the sanitizing source 3.

In particular, the app (via the device 38) or the switch 39 sends a command to the control device 5; after a preset exit time (which can be set through the app to allow exit from the room), the control device 5 sends an indirect command to the sanitizing source 3. The third relay 33 closes to indicate that sanitization is on.

During the exit time, the first relay 31 (for the visual signalling LED 17) and the second relay 32 (for the sound signal 19) are active, e.g., intermittently. Optionally, however, the output of the sound signal 19 can be silenced via the app.

When the exit time is over, the second relay 32 (the sound signal 19) switches off and the first relay 31 (the visual signalling LED 17) flashes to indicate that sanitization is in progress.
- Disabling of sanitization: the sanitizing source 3 is disabled via the app (the device 38) or the switch 39, or by intervention of the presence sensor 13 or of one of the other sensors 15.

Once the corresponding signal has been received, the control device 5 opens the third relay 33 and the first relay 31 to indicate that the sanitization is switched off.

This step measures the value of the current in the load (the sanitizing source 3) by means of the amperometric sensor 35:
1) if the measured current is lower than a threshold value preset in the control device 5, this means that the sanitizing source 3 (e.g., the violet light source) is switched on in a non-hazardous manner, or is switched off;
2) if the measured current is higher than the preset threshold value (the sanitizing source 3 is activated above the threshold value), the control device 5 sends a command for reducing or switching off the power of the sanitizing source 3 to the respective driver 23. The sanitizing source 3, following this command, goes back to the pre-sanitization state (for example, a state where the sanitizing source 3 is switched off or operates below the threshold value). If the measured current still does not fall below the threshold value (which is a safe value for the exposure of users), the fourth relay 34 intervenes by cutting off the power to the driver 23 of the sanitizing source 3.

The presence sensor 13 and the other sensors 15 define a safety chain, and the control device 5 is configured to give consent to the use of the sanitizing source 3 only if the safety chain is closed, that is, if all safety criteria are met.

If, then, during operation in the sanitizing mode (sanitizing source 3 on), the control device 5 detects an interruption of the safety chain, e.g., the presence of a person detected by the presence sensor 13 or the opening of a door detected by one of the sensors 15, the control device 5 stops the sanitization by switching off the sanitizing source (or reducing its power supply below the safety threshold value).

In the event of a malfunction of the wireless communication system (or, in any case, of the communication system, which can also be wired, between the control device 5 and the presence sensor 13 and the other sensors 15), the control device 5 is still able to ensure user safety, because the amperometric sensor 35 monitors the current flowing in the sanitizing source 3, and if the source is switched on and a malfunction is detected in the communication system, it disables the sanitizing source 3.

Additional safety features are the alerts sent during sanitization by the visual signalling LED 17 and the sound signal 19.

The following settings are available through the app:
- activation of the safety chain operation test;
- on/off setting of the sound signal;
- self-calibration of the current threshold value in the sanitizing source;
- setting of the exit time prior to the switching on of the sanitizing source;
- setting of the automatic sanitization switching off time.

The control device 5 allows a shared line to be used safely for all operating modes: in fact, the control device 5 discerns the operating mode actually in progress on the basis of the absorption detected by the amperometric sensor 5: if the amperometric sensor 35 detects a current higher than the threshold value, the lighting system 1 operates in the sanitizing mode (for example, night mode); if it detects a current lower than the threshold value, the lighting system 1 operates in the daytime mode (without sanitization). In the event of an interruption of the safety chain, as described above, the control device 5 intervenes and switches off the sanitizing source or reduces its power supply below the safety threshold value.

## Claims

1. A control device (5) for controlling a lighting system (1) with sanitizing function, comprising: a printed circuit board (10) connectable to at least one lamp (2) of the lighting system (1) provided with at least one sanitizing source (3); at least one presence sensor (13) connected to the board (10) by a communication system (37), for example a wireless communication system; wherein the board (10) is configured so as to disable the sanitizing source (3) or to reduce the power supply to the sanitizing source below a threshold value when the presence sensor (13) detects the presence of users;
**characterised by**
- an amperometric sensor (35) connected to the sanitizing source (3) to measure the current in the sanitizing source (3);
- the board is configured so as to disable the sanitizing source (3) or to reduce the power supply to the sanitizing source below a threshold value also when a malfunction is detected in the communication system (37) and the amperometric sensor (35) detects in the sanitizing source (3) a current greater than the threshold value.

2. The control device according to claim 1, comprising at least one visual signalling LED (17) and/or a sound signal (19); and wherein the board (10) is configured so as to activate the visual signalling LED (17) and/or the sound signal (19) when the sanitizing source (3) is active.

3. The control device according to claim 1 or 2, wherein the board (10) is configured so as to measure the current in the sanitizing source (3) by means of the amperometric sensor (35); and if the measured current is lower than the threshold value, the control device (5) does not operate on the sanitizing source (3); if the measured current is higher than the threshold value, the control device (5) sends a command for reducing or switching off the power of the sanitizing source (3) to bring the sanitizing source (3) back to a pre-sanitization operation mode; and if the measured current still remains higher than the threshold value, the control device cuts off the power to the sanitizing source (3).

4. The control device according to one of the preceding claims, wherein the board (10) comprises: a supply input (11) for connection to a power supply; a sensor input (12) connected to the presence sensor (13); an outer sensors input (14) for connection to further sensors (15); a current measure input (24) for measuring the current in the sanitizing source (3); a transformer input (25) for connection to an amperometric transformer (26) for measuring the primary current in a driver (23) of the sanitizing source (3) .

5. The control device according to one of the preceding claims, wherein the board (10) comprises: a first relay contact output (16) for connection to the visual signalling LED (17); a second relay contact output (18) for connection to the sound signal (19); a third relay contact output (20) for sanitization in progress; a fourth relay contact output (22) for connection in series to a driver (23) of the sanitizing source (3).

6. The control device according to one of the preceding claims, wherein the communication system (37) is a wireless communication system communicating with a remote device (38), for example a smartphone or another similar device, on which a dedicated application is loaded providing a user interface with the control device (5).

7. The control device according to one of the preceding claims, wherein the board (10) is configured so as to provide the consent to switch on the sanitizing source (3), following a switch-on command sent by a user via a remote device (38) or a switch (39), only if no users are detected by the presence sensor (13) and/or by the other sensors (15).

8. The control device according to claim 7, wherein the board (10) is configured to provide the consent to switch on the sanitizing source (3) after a preset exit time.

9. A lighting system comprising at least one light source emitting substantially white visible light, at least one violet light source with sanitizing capability, and at least one UV source; and a control device connected to said sources; **characterized in that** the control device is a control device according to one of claims 1 to 8.

10. A control method for controlling a lighting system (1) with sanitizing function having at least one lamp (2) provided with at least one sanitizing source (3) ; the control method comprising the steps of: detecting the presence of users in the environment in which the lighting system (1) is installed by means of at least one presence sensor (13) connected via a communication system (37), for example a wireless communication system, to a control device (5) ; disabling the sanitizing source (3) or reducing the power supply to the sanitizing source below a threshold value when the presence sensor (13) detects the presence of users;
**characterised by**
- monitoring by means of an amperometric sensor (35) connected to the sanitizing source (3) the current in the sanitizing source (3);
- disabling the sanitizing source (3) or reducing the power supply to the sanitizing source below a threshold value also when a malfunction is detected in the communication system (37) and the amperometric sensor (35) detects in the sanitizing source (3) a current greater than the threshold value.

11. The control method according to claim 10, comprising the steps of emitting a luminous and/or sound alarm when the sanitizing source (3) is active.

12. The control method according to claim 10 or 11, comprising the steps of: measuring the current in the sanitizing source (3) by means of the amperometric sensor (35); if the measured current is lower than the threshold value, the operation of the sanitizing source (3) is not changed; if the measured current is higher than the threshold value, a command for reducing or switching off the power of the sanitizing source (3) is sent to bring the sanitizing source (3) back to a pre-sanitization operation mode; and if the measured current still remains higher than the threshold value, the power to the sanitizing source (3) is cut off.

13. The control method according to one of claims 10 to 12, wherein the control device (5) provides the consent to switch on the sanitizing source (3), following a switch-on command sent by a user via a remote device (38) or a switch (39), only if no users are detected by the presence sensor (13) and/or by the other sensors (15).

14. The control method according to claim 13, wherein the consent to switch on the sanitizing source (3) is provided after a preset exit time.

## Patentansprüche

1. Steuervorrichtung (5) zum Steuern eines Beleuchtungssystems (1) mit Desinfektionsfunktion, umfassend: eine Platine (10), die mit mindestens einer mit mindestens einer Desinfektionsquelle (3) versehenen Lampe (2) des Beleuchtungssystems (1) verbindbar ist; mindestens einen Anwesenheitssensor (13), der über ein Kommunikationssystem (37), wie beispielsweise ein drahtloses Kommunikationssystem, mit der Platine (10) verbunden ist; wobei die Platine (10) konfiguriert ist, die Desinfektionsquelle (3) zu deaktivieren oder die Stromzufuhr zu der Desinfektionsquelle unter einen Schwellenwert zu reduzieren, wenn der Anwesenheitssensor (13) die Anwesenheit von Benutzern detektiert;
**gekennzeichnet durch**:
- einen amperometrischen Sensor (35), der mit der Desinfektionsquelle (3) verbunden ist, um den Strom in der Desinfektionsquelle (3) zu messen;
- dass die Platine konfiguriert ist, die Desinfektionsquelle (3) zu deaktivieren oder die Stromzufuhr der Desinfektionsquelle unter einen Schwellenwert zu reduzieren, auch wenn eine Fehlfunktion in dem Kommunikationssystem (37) detektiert wird und der amperometrische Sensor (35) in der Desinfektionsquelle (3) einen größeren Strom als der Schwellenwert detektiert.

2. Steuervorrichtung nach Anspruch 1, umfassend mindestens eine optische Signalisierungs-LED (17) und/oder ein Tonsignal (19); wobei die Platine (10) konfiguriert ist, die optische Signalisierungs-LED (17) und/oder das Tonsignal (19) zu aktivieren, wenn die Desinfektionsquelle (3) aktiv ist.

3. Steuervorrichtung nach Anspruch 1 oder 2, wobei die Platine (10) konfiguriert ist, den Strom in der Desinfektionsquelle (3) mithilfe des amperometrischen Sensors (35) zu messen; und, wenn der gemessene Strom niedriger ist als der Schwellenwert, die Steuervorrichtung (5) die Desinfektionsquelle (3) nicht betreibt; wenn der gemessene Strom höher ist als der Schwellenwert, die Steuervorrichtung (5) einen Befehl zum Reduzieren oder Abschalten des Stroms der Desinfektionsquelle (3) sendet, um die Desinfektionsquelle (3) in einen Betriebsmodus vor der Desinfektion zurückzuversetzen; und, wenn sich der gemessene Strom immer noch über dem Schwellenwert befindet, die Steuervorrichtung die Stromzufuhr zu der Desinfektionsquelle (3) unterbricht.

4. Steuervorrichtung nach einem der vorstehenden Ansprüche, wobei die Platine (10) umfasst: einen Versorgungseingang (11) zur Verbindung mit einer Stromversorgung; einen Sensoreingang (12), der mit dem Anwesenheitssensor (13) verbunden ist; einen Eingang für äußere Sensoren (14) zur Verbindung mit weiteren Sensoren (15); einen Strommesseingang (24) zum Messen des Stroms in der Desinfektionsquelle (3); einen Wandlereingang (25) zum Anschluss an einen amperometrischen Wandler (26) zum Messen des Primärstroms in einer Antriebsvorrichtung (23) der Desinfektionsquelle (3).

5. Steuervorrichtung nach einem der vorstehenden Ansprüche, wobei die Platine (10) umfasst: einen ersten Relaiskontaktausgang (16) zur Verbindung mit der optischen Signalisierungs-LED (17); einen zweiten Relaiskontaktausgang (18) zur Verbindung mit dem Tonsignal (19); einen dritten Relaiskontaktausgang (20) für Desinfektion im Gange; einen vierten Relaiskontaktausgang (22) zur Verbindung mit einer Antriebsvorrichtung (23) der Desinfektionsquelle (3) in Reihe.

6. Steuervorrichtung nach einem der vorstehenden Ansprüche, wobei das Kommunikationssystem (37) ein drahtloses Kommunikationssystem ist, das mit einer Fernbedienungsvorrichtung (38), wie beispielsweise einem Smartphone oder einer anderen ähnlichen Vorrichtung, kommuniziert, auf das bzw. die eine spezielle Anwendung geladen ist, die eine Benutzerschnittstelle mit der Steuervorrichtung (5) bereitstellt.

7. Steuervorrichtung nach einem der vorstehenden Ansprüche, wobei die Platine (10) konfiguriert ist, die Zustimmung zum Einschalten der Desinfektionsquelle (3) nach einem von einem Benutzer über eine Fernbedienungsvorrichtung (38) oder einen Schalter (39) gesendeten Einschaltbefehl nur dann zu erteilen, wenn durch den Anwesenheitssensor (13) und/oder durch die anderen Sensoren (15) keine Benutzer detektiert werden.

8. Steuervorrichtung nach Anspruch 7, wobei die Platine (10) konfiguriert ist, die Zustimmung zum Einschalten der Desinfektionsquelle (3) nach einer voreingestellten Endezeit zu erteilen.

9. Beleuchtungssystem, umfassend mindestens eine Lichtquelle, die im Wesentlichen weißes sichtbares Licht aussendet, mindestens eine violette Lichtquelle mit Desinfektionsfähigkeit und mindestens eine UV-Quelle; und eine Steuervorrichtung, die mit den Quellen verbunden ist; **dadurch gekennzeichnet, dass** die Steuervorrichtung eine Steuervorrichtung nach einem der Ansprüche 1 bis 8 ist.

10. Steuerverfahren zum Steuern eines Beleuchtungssystems (1) mit Desinfektionsfunktion, das mindestens eine mit mindestens einer Desinfektionsquelle (3) versehene Lampe (2) aufweist; wobei das Steuerverfahren die Schritte umfasst: Detektieren der Anwesenheit von Benutzern in der Umgebung, in der das Beleuchtungssystem (1) installiert ist, mithilfe mindestens eines Anwesenheitssensors (13), der über ein Kommunikationssystem (37), wie beispielsweise ein drahtloses Kommunikationssystem, mit einer Steuervorrichtung (5) verbunden ist; Deaktivieren der Desinfektionsquelle (3) oder Reduzieren der Stromzufuhr zu der Desinfektionsquelle unter einen Schwellenwert, wenn der Anwesenheitssensor (13) die Anwesenheit von Benutzern detektiert;
**gekennzeichnet durch**:
- Überwachen des Stroms in der Desinfektionsquelle (3) mithilfe eines amperometrischen Sensors (35), der mit der Desinfektionsquelle (3) verbunden ist;
- Deaktivieren der Desinfektionsquelle (3) oder Reduzieren der Stromzufuhr der Desinfektionsquelle unter einen Schwellenwert, auch wenn eine Fehlfunktion in dem Kommunikationssystem (37) detektiert wird und der amperometrische Sensor (35) in der Desinfektionsquelle (3) einen größeren Strom als der Schwellenwert detektiert.

11. Steuerverfahren nach Anspruch 10, umfassend die Schritte des Aussendens eines Leucht- und/oder Tonalarms, wenn die Desinfektionsquelle (3) aktiv ist.

12. Steuerverfahren nach Anspruch 10 oder 11, das die Schritte umfasst: Messen des Stroms in der Desinfektionsquelle (3) mithilfe des amperometrischen Sensors (35); wenn der gemessene Strom niedriger ist als der Schwellenwert, kein Verändern des Betriebs der Desinfektionsquelle (3); wenn der gemessene Strom höher ist als der Schwellenwert, Senden eines Befehls zum Reduzieren oder Abschalten des Stroms der Desinfektionsquelle (3), um die Desinfektionsquelle (3) in einen Betriebsmodus vor der Desinfektion zurückzuversetzen; und, wenn der gemessene Strom weiterhin über dem Schwellenwert verbleibt, Unterbrechen der Stromzufuhr zu der Desinfektionsquelle (3) .

13. Verfahren nach einem der Ansprüche 10 bis 12, wobei die Steuervorrichtung (5) die Zustimmung zum Einschalten der Desinfektionsquelle (3) nach einem von einem Benutzer über eine Fernbedienungsvorrichtung (38) oder einen Schalter (39) gesendeten Einschaltbefehl nur dann erteilt, wenn durch den Anwesenheitssensor (13) und/oder durch die anderen Sensoren (15) keine Benutzer detektiert werden.

14. Steuerverfahren nach Anspruch 13, wobei die Zustimmung zum Einschalten der Desinfektionsquelle (3) nach einer voreingestellten Endezeit erteilt wird.

## Revendications

1. Dispositif de commande (5) pour commander un système d'éclairage (1) avec fonction de désinfection, comprenant : une carte de circuit imprimé (10) connectable à au moins une lampe (2) du système d'éclairage (1) pourvue d'au moins une source de désinfection (3) ; au moins un capteur de présence (13) connecté à la carte (10) par un système de communication (37), par exemple un système de communication sans fil ;
dans lequel la carte (10) est configurée pour désactiver la source de désinfection (3) ou pour réduire l'alimentation électrique de la source de désinfection en dessous d'une valeur seuil lorsque le capteur de présence (13) détecte la présence d'utilisateurs ;
**caractérisé par**
- un capteur ampérométrique (35) connecté à la source de désinfection (3) pour la mesure du courant dans la source de désinfection (3) ;
- la carte est configurée pour désactiver la source de désinfection (3) ou pour réduire l'alimentation électrique de la source de désinfection en dessous d'une valeur seuil également lorsqu'un dysfonctionnement est détecté dans le système de communication (37) et que le capteur ampérométrique (35) détecte dans la source de désinfection (3) un courant supérieur à la valeur seuil.

2. Dispositif de commande selon la revendication 1, comprenant au moins une DEL de signalisation visuelle (17) et/ou un signal sonore (19) ; et dans lequel la carte (10) est configurée de manière à activer la DEL de signalisation visuelle (17) et/ou le signal sonore (19) lorsque la source de désinfection (3) est active.

3. Dispositif de commande selon la revendication 1 ou 2, dans lequel la carte (10) est configurée de manière à mesurer le courant dans la source de désinfection (3) à l'aide du capteur ampérométrique (35) et ; si le courant mesuré est inférieur à la valeur seuil, le dispositif de commande (5) n'agit pas sur la source de désinfection (3) ; si le courant mesuré est supérieur à la valeur seuil, le dispositif de commande (5) envoie une instruction pour réduire ou mettre à l'arrêt la puissance de la source de désinfection (3) pour ramener la source de désinfection (3) à un mode de fonctionnement pré-désinfection ; et si le courant mesuré reste supérieur à la valeur seuil, le dispositif de commande coupe l'alimentation de la source de désinfection (3).

4. Dispositif de commande selon l'une des revendications précédentes, dans lequel la carte (10) comprend : une entrée d'alimentation (11) pour la connexion à une alimentation électrique ; une entrée de capteur (12) connectée au capteur de présence (13) ; une entrée de capteurs extérieurs (14) pour la connexion à des capteurs supplémentaires (15) ; une entrée de mesure de courant (24) pour la mesure du courant dans la source de désinfection (3) ; une entrée de transformateur (25) pour la connexion à un transformateur ampérométrique (26) pour la mesure du courant primaire dans un pilote (23) de la source de désinfection (3).

5. Dispositif de commande selon l'une des revendications précédentes, dans lequel la carte (10) comprend : une première sortie de contact de relais (16) pour la connexion à la DEL de signalisation visuelle (17) ; une deuxième sortie de contact de relais (18) pour la connexion au signal sonore (19) ; une troisième sortie de contact de relais (20) pour la désinfection en cours ; une quatrième sortie de contact de relais (22) pour la connexion en série à un pilote (23) de la source de désinfection (3).

6. Dispositif de commande selon l'une des revendications précédentes, dans lequel le système de communication (37) est un système de communication sans fil communiquant avec un dispositif distant (38), par exemple un téléphone intelligent ou un autre dispositif similaire, sur lequel est chargée une application dédiée fournissant une interface utilisateur avec le dispositif de commande (5).

7. Dispositif de commande selon l'une des revendications précédentes, dans lequel la carte (10) est configurée de manière à donner l'autorisation de mettre en marche la source de désinfection (3) suite à une instruction de mise en marche envoyée par un utilisateur via un dispositif distant (38) ou un commutateur (39) uniquement si aucun utilisateur n'est détecté par le capteur de présence (13) et/ou par les autres capteurs (15).

8. Dispositif de commande selon la revendication 7, dans lequel la carte (10) est configurée de manière à donner l'autorisation de mettre en marche la source de désinfection (3) après un temps de sortie prédéfini.

9. Système d'éclairage comprenant au moins une source de lumière émettant une lumière visible sensiblement blanche, au moins une source de lumière violette avec une capacité de désinfection, et au moins une source UV ; et un dispositif de commande connecté auxdites sources ; **caractérisé en ce que** le dispositif de commande est un dispositif de commande selon l'une des revendications 1 à 8.

10. Procédé de commande pour commander un système d'éclairage (1) avec fonction de désinfection doté d'au moins une lampe (2) pourvue d'au moins une source de désinfection (3) ; le procédé de commande comprenant les étapes suivantes :
détecter la présence d'utilisateurs dans l'environnement où le système d'éclairage (1) est installé au moyen d'au moins un capteur de présence (13) connecté via un système de communication (37), par exemple un système de communication sans fil, à un dispositif de commande (5) ;
désactiver la source de désinfection (3) ou réduire l'alimentation électrique de la source de désinfection en dessous d'une valeur seuil lorsque le capteur de présence (13) détecte la présence d'utilisateurs ;
**caractérisé par**
- à l'aide d'un capteur ampérométrique (35) connecté à la source de désinfection (3), la surveillance du courant dans la source de désinfection (3) ;
- la désactivation de la source de désinfection (3) ou la réduction de l'alimentation électrique de la source de désinfection en dessous d'une valeur seuil lorsqu'un dysfonctionnement est détecté dans le système de communication (37) et que le capteur ampérométrique (35) détecte dans la source de désinfection (3) un courant supérieur à la valeur seuil.

11. Procédé de commande selon la revendication 10, comprenant l'étape suivante d'émission d'une alarme lumineuse et/ou sonore lorsque la source de désinfection (3) est active.

12. Procédé de commande selon la revendication 10 ou 11, comprenant les étapes suivantes : mesurer le courant dans la source de désinfection (3) à l'aide du capteur ampérométrique (35) ; si le courant mesuré est inférieur à la valeur seuil, le fonctionnement de la source de désinfection (3) n'est pas modifié ; si le courant mesuré est supérieur à la valeur seuil, une instruction de réduction ou de mise à l'arrêt de la puissance de la source de désinfection (3) est envoyée pour ramener la source de désinfection (3) à un mode de fonctionnement pré-désinfection ; et si le courant mesuré reste supérieur à la valeur seuil, l'alimentation de la source de désinfection (3) est coupée.

13. Procédé de commande selon l'une des revendications 10 à 12, dans lequel le dispositif de commande (5) donne l'autorisation de mettre en marche la source de désinfection (3) suite à une instruction de mise en marche envoyée par un utilisateur via un dispositif distant (38) ou un commutateur (39) uniquement si aucun utilisateur n'est détecté par le capteur de présence (13) et/ou par les autres capteurs (15).

14. Procédé de commande selon la revendication 13, dans lequel l'autorisation de mettre en marche la source de désinfection (3) est donnée après un temps de sortie prédéfini.
